(19) **Europäisches Patentamt / European Patent Office / Office européen des brevets**

(11) **EP 3 321 408 B1**

(12) **EUROPEAN PATENT SPECIFICATION**

(45) Date of publication and mention
of the grant of the patent:
**11.11.2020 Bulletin 2020/46**

(21) Application number: **16821441.9**

(22) Date of filing: **06.07.2016**

(51) Int Cl.:
*D04H 3/14* (2012.01)  *C08K 5/20* (2006.01)
*C08L 23/08* (2006.01)  *C08L 23/12* (2006.01)
*C08L 23/14* (2006.01)  *D04H 3/007* (2012.01)
*D04H 3/16* (2006.01)  *A61F 13/51* (2006.01)
*D04H 3/147* (2012.01)  *A41B 13/04* (2006.01)

(86) International application number:
**PCT/JP2016/070052**

(87) International publication number:
**WO 2017/006972 (12.01.2017 Gazette 2017/02)**

(54) **SPUNBOND NONWOVEN FABRIC AND HYGIENIC MATERIAL**

VERSPONNENER VLIESSTOFF UND HYGIENEMATERIAL

NON-TISSÉ FILÉ-LIÉ ET MATÉRIAU HYGIÉNIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.07.2015 JP 2015135490**

(43) Date of publication of application:
**16.05.2018 Bulletin 2018/20**

(73) Proprietor: **Mitsui Chemicals, Inc.**
**Minato-ku**
**Tokyo 105-7122 (JP)**

(72) Inventors:
• **MATSUBARA, Akio**
  **Sodegaura-shi**
  **Chiba 299-0265 (JP)**
• **SUZUKI, Kenichi**
  **Sodegaura-shi**
  **Chiba 299-0265 (JP)**

(74) Representative: **Hoffmann Eitle**
**Patent- und Rechtsanwälte PartmbB**
**Arabellastraße 30**
**81925 München (DE)**

(56) References cited:
EP-A1- 3 165 656    WO-A1-2006/065663
WO-A1-2009/145105    WO-A1-2014/050965
WO-A1-2014/115401    JP-A- 2000 045 165
JP-A- 2002 531 721    JP-A- 2009 538 394
JP-A- 2010 168 713    JP-A- 2012 012 759
JP-A- 2013 032 607    US-A1- 2005 165 173
US-A1- 2010 124 864    US-A1- 2011 238 027

• **Exxon Mobil: "Product Name: ACHIEVE(TM) PP POLYMERS", , 4 April 2018 (2018-04-04), XP055548106, Retrieved from the Internet: URL:http://www.msds.exxonmobil.com/IntApps /psims/SearchResults.aspx [retrieved on 2019-01-28]**

**Description**

TECHNICAL FIELD

The present disclosure relates to a spunbond nonwoven fabric and a hygienic material.

BACKGROUND ART

Nonwoven fabrics have been used recently in various applications broadly owing to their superior air permeability and flexibility. Consequently, it has been required that nonwoven fabrics have various characteristics corresponding to the respective applications, and that such characteristics are improved.

For example, a nonwoven fabric to be used as an absorbent article, such as a disposable diaper or a sanitary napkin, a hygiene mask, medical gauze, or base fabric for poultice is required to be water resistant and superior in moisture permeability. Further, depending on a site where it is to be used, it is required to have extensibility and flexibility, and further, it is required to have superior heat-sealability for favorable processability.

As one method to impart extensibility to a nonwoven fabric, a method of using a thermoplastic elastomer as a raw material for a spunbond nonwoven fabric has been proposed (for example, refer to Japanese National-Phase Publication (JP-A) No. H07-503502). However, in this case, there is a problem in that, when the content of a thermoplastic elastomer is high, surface stickiness occurs, and flexibility and tactile feel of a nonwoven fabric are impaired.

Further, as a nonwoven fabric contained in a composite nonwoven fabric, a coherent extensible nonwoven fabric which contains an ethylene polymer and a propylene polymer and is superior in extensibility has been proposed (for example, refer to Japanese Patent No. 3798018).

As a nonwoven fabric having flexibility, a spunbond nonwoven fabric composed of a composition containing two or more polypropylenes having melting points different from each other, and a specific fatty acid amide, has been proposed (for example, refer to WO 2014/050965 A1).

As a spunbond nonwoven fabric which is superior in stretchability and tactile feel, and is suitable for a hygienic material, a spunbond nonwoven fabric using a thermoplastic polyurethane elastomer prepared by adding ethylenebis (oleic amide) and/or crosslinked organic fine particles to a thermoplastic polyurethane elastomer to attain a hardness in a range of from 75 to 85 has been proposed (for example, refer to Japanese Patent Application Laid-Open (JP-A) No. 2015-71854). Further relevant prior art is US 2010/0124864 A1.

SUMMARY OF INVENTION

TECHNICAL PROBLEM

A coherent extensible nonwoven fabric according to Japanese Patent No. 3798018 was not adequate for use as an independent nonwoven fabric in terms of stretching processing suitability, extensibility, and feeling, although an embodiment of a composite nonwoven fabric having a second extensible layer was anticipated. A spunbond nonwoven fabric according to WO 2014/050965 A1 is superior in flexibility, but there is still room for improvement in heat-sealability.

As for a nonwoven fabric according to JP-A No. 2015-71854, although the stretchability is improved, and deterioration in feeling due to fiber stickiness is suppressed, there is still room for improvement with respect to extensibility, heat-sealability, and stretching processing suitability.

An object of an embodiment of the present invention is to provide a spunbond nonwoven fabric superior in low temperature heat-sealability and stretching processing suitability, and a hygienic material using the spunbond nonwoven fabric.

SOLUTION TO PROBLEM

Means for solving the above problems includes a spunbond nonwoven fabric according to the subject-matter of claim 1, and a hygienic material comprising the spunbond nonwoven fabric. Particular embodiments are further defined by the dependent claims.

ADVANTAGEOUS EFFECTS OF INVENTION

According to an embodiment of the invention, a spunbond nonwoven fabric superior in low temperature heat-sealability and stretching processing suitability, and a hygienic material using the spunbond nonwoven fabric are provided.

BRIEF DESCRIPTION OF DRAWINGS

Fig. 1 is a schematic diagram of a gear stretching device.
Fig. 2 is a schematic diagram of an open-system spunbond method in which melt spun continuous fibers are stretched while being cooled in the air.
Fig. 3 is a schematic diagram of a closed system spunbond method.

DESCRIPTION OF EMBODIMENTS

An embodiment of the invention (hereinafter also referred to as "the embodiment") will be described below.
However, the invention is not limited to the following embodiment. In the following embodiment, its constituent (including constituent step) is not essential, unless it is so specified, or unless it is clear that it is essential in principle. Similarly, a numerical value and a range thereof do not restrict in any way the invention.
The term "step" includes herein not only an independent step, but also a step which may not be clearly separated from another step, insofar as an intended function of the step can be attained.
A numerical range expressed by "x to y" includes herein the values of x and y in the range as the minimum and maximum values, respectively.
In referring herein to a content of a component in a composition, when plural substances exist corresponding to a component in the composition, the content means, unless otherwise specified, the total amount of the plural substances existing in the composition.

<Spunbond Nonwoven Fabric>

A spunbond nonwoven fabric of the embodiment is a spunbond nonwoven fabric, consisting of a composition including a propylene homopolymer having a melting point of 140°C or higher, an ethylene homopolymer having a density of 0.941-0.970 g/cm$^3$, and at least one polymer selected from the group consisting of a polymer shown in the following (I) and a polymer shown in the following (II); wherein the total content of the polymer shown in the following (I) and the polymer shown in the following (II) in the composition is in a range of from 5% by mass to 30% by mass with respect to the total amount of the composition:

(I) a random copolymer of propylene and at least one selected from ethylene and a C4-C20 $\alpha$-olefin, that has a melting of $\geq$ 130°C, and that has a melt flow rate of 30-70 g/10 min, and
(II) a propylene homopolymer, which has a melting point less than 120°C, and satisfies the following (a) to (f):

(a) [mmmm] = 20 mol% to 60 mol%
(b) [rrrr]/(1-[mmmm]) $\leq$ 0.1
(c) [rmrm] > 2.5 mol%
(d) [mm]$\times$[rr]/[mr]$^2$ $\leq$ 2.0
(e) weight-average molecular weight (Mw) = 10,000 to 200,000
(f) molecular weight distribution (Mw/Mn) < 4,

wherein, in (a) to (d), [mmmm] represents a meso pentad fraction, [rrrr] represents a racemic pentad fraction, [rmrm] represents a racemic meso racemic meso pentad fraction, and [mm], [rr] and [mr] each represent a triad fraction.

A spunbond nonwoven fabric of the embodiment contains, in addition to a propylene homopolymer having a relatively high melting point and an ethylene homopolymer, further a polymer selected from the group consisting of a random copolymer of an $\alpha$-olefin having a specific number of carbon atoms and propylene, and a propylene homopolymer having a relatively low melting point and specific meso pentad fraction and racemic pentad fraction, in an amount of from 5% by mass to 30% by mass with respect to the total amount of the composition to be used as a raw material for the nonwoven fabric. As a consequence, an obtained spunbond nonwoven fabric acquires superior low temperature heat-sealability and stretching processing suitability owing to the physical properties of the composition. A spunbond nonwoven fabric of the embodiment has further advantages in terms of superior extensibility and flexibility.
The superior "stretching processing suitability" of a spunbond nonwoven fabric of the embodiment may be confirmed by the fact that an embossment survival rate is high after stretching processing to impart flexibility to a spunbond nonwoven fabric and breakage of a spunbond nonwoven fabric does not occur in stretching processing.
Presence of the respective components in a composition included in a spunbond nonwoven fabric of the embodiment may be confirmed appropriately by a method known in the art.

The meso pentad fraction [mmmm], racemic pentad fraction [rrrr], and racemic meso racemic meso pentad fraction [rmrm] as well as triad fractions [mm], [rr], and [mr] of a propylene homopolymer having a melting point less than 120°C as a polymer shown in (II) may be calculated according to the method proposed by A. Zambelli, et al. "Macromolecules, 6, 925 (1973)" as described in detail below.

The density of an ethylene homopolymer contained in a spunbond nonwoven fabric of the embodiment is in a range of from 0.941 g/cm³ to 0.970 g/cm³ from the viewpoint of improvement of the extensibility and flexibility of a spunbond nonwoven fabric to be obtained. Further, when a high density polyethylene is contained, improvement of the strength of a spunbond nonwoven fabric may also be expected.

The content of an ethylene homopolymer with respect to the total amount of the composition is preferably from 1.0% by mass to 10.0% by mass.

When the content of an ethylene homopolymer is in the above range, the extensibility of a spunbond nonwoven fabric to be obtained is improved.

A composition included in a spunbond nonwoven fabric of the embodiment contains preferably a C15-C22 fatty acid amide. When the composition contains the fatty acid amide, the fatty acid amide is adsorbed on a fiber surface of a spunbond nonwoven fabric formed with the composition, so as to modify the fiber surface. As the result, the extensibility and flexibility of a nonwoven fabric are improved.

The content of a fatty acid amide is preferably from 0.1% by mass to 5.0% by mass with respect to the total amount of the composition.

The content of a propylene homopolymer having a melting point of 140°C or higher in a composition included in a spunbond nonwoven fabric of the embodiment is preferably from 55.0% by mass to 89.9% by mass with respect to the total amount of the composition.

When the content of a propylene homopolymer having a melting point of 140°C or higher in a composition included in a spunbond nonwoven fabric of the embodiment is in the above range, the strength properties of a nonwoven fabric may be kept in a favorable range, and a flexible nonwoven fabric having a low basis weight may be obtained.

A random copolymer of propylene and an α-olefin shown in (I) above contained in a composition for forming a spunbond nonwoven fabric of the embodiment is preferably a random copolymer of propylene and ethylene, or a random copolymer of propylene, ethylene, and 1-butene. When an α-olefin contained in a copolymer includes ethylene, the extensibility of an obtained spunbond nonwoven fabric may be improved.

A spunbond nonwoven fabric of the embodiment may be produced by an ordinary method using a composition described below in detail.

There is no particular restriction on the basis weight of a spunbond nonwoven fabric of the embodiment. A nonwoven fabric of the embodiment has preferably a basis weight of, ordinarily, 30 g/m² or less from the viewpoint of securement of both flexibility and strength, more preferably 28 g/m² or less, further preferably 25 g/m² or less, and most preferably in a range of from 20 g/m² to 5 g/m². When a spunbond nonwoven fabric of the embodiment is applied to a hygienic material described below, or the like, the basis weight of a spunbond nonwoven fabric is preferably in a range of from 19 g/m² to 5 g/m².

A fiber included in a spunbond nonwoven fabric has preferably a fiber diameter of, ordinarily, 50 μm or less, more preferably 40 μm or less, further preferably 30 μm or less, and most preferably 20 μm or less. Although a smaller fiber diameter gives better flexibility of a nonwoven fabric, from the viewpoint of handling property, production suitability, and suppression of fuzzing of an obtained nonwoven fabric, the fiber diameter is preferably 10 μm or more.

[Physical Properties of Spunbond Nonwoven Fabric]

Preferable physical properties of a spunbond nonwoven fabric of the embodiment will be listed below.

(Heat-Sealability)

Heat-sealability is one of the physical properties of a spunbond nonwoven fabric of the embodiment. As for the heat-sealability of a spunbond nonwoven fabric, when two sheets of a nonwoven fabric are laid together and heat-sealed by a heat-seal tester, it is preferable that heat-sealing is possible at 180°C or lower, and more preferable that heat-sealing is possible at 160°C or lower.

Further, it is preferable that burn, namely discoloration by heating, is suppressed in heat-sealing under the above conditions.

Although the tensile peel strength of heat-sealed two sheets of a spunbond nonwoven fabric may be selected appropriately corresponding to an intended use, generally it is preferably 0.05 N/20 mm or more, and more preferably 0.1 N/20 mm or more.

(Embossment Survival Rate)

An embossment survival rate is one of the physical properties of a spunbond nonwoven fabric of the embodiment. The embossment survival rate of a spunbond nonwoven fabric after stretching processing is preferably 40% or more, more preferably 50% or more, and further preferably 70% or more. When the embossment survival rate after stretching processing is 40% or more, the tactile feel of a spunbond nonwoven fabric is improved. An embossment survival rate may be measured by a method used in Examples described below.

(Flexibility)

Flexibility is one of the physical properties of a spunbond nonwoven fabric of the embodiment. The flexibility of a spunbond nonwoven fabric has a large influence on sense of use of a nonwoven fabric. With respect to flexibility, there are a flexibility according to sensory evaluation by touch, and a bending resistance. A bending resistance may be measured according to JIS L 1096, 8.19.1 [A method (45° cantilever method)] as described in detail in Examples.

(Maximum Strength and Maximum Elongation)

Maximum elongation and maximum load are respectively one of preferable physical properties of a spunbond nonwoven fabric of the embodiment. The maximum elongation of a spunbond nonwoven fabric of the embodiment in at least one direction is preferably 70% or higher, more preferably 100% or higher, and further preferably 140% or higher. Further, a spunbond nonwoven fabric having a character substantially free of elastic recovery is preferable.

A maximum elongation and a maximum strength of a spunbond nonwoven fabric may be measured according to JIS L 1906, 6.12.1 [A method] as described in detail in Examples.

A spunbond nonwoven fabric of the embodiment may be produced by an ordinary method using one or two or more kinds of compositions described below in detail.

[Composition]

A composition included in a spunbond nonwoven fabric of the embodiment contains as described above a propylene homopolymer having a melting point of 140°C or higher (hereinafter also referred to as "specific polypropylene"), an ethylene homopolymer, and at least one polymer selected from the group consisting of a polymer shown in (I) above and a polymer shown in (II) above (hereinafter also referred to as "specific polymer").

The total content of the specific polymer is from 5% by mass to 30% by mass with respect to the total amount of the composition as 100% by mass.

The content of the specific polymer is preferably from 10% by mass to 30% by mass with respect to the total amount of the composition, and more preferably from 10% by mass to 25% by mass.

When a spunbond nonwoven fabric of the embodiment contains at least one polymer selected from the group consisting of polymers shown in (I) and (II) above, it can acquire excellent stretching processing suitability, while securing favorable flexibility and extensibility.

The content of a propylene homopolymer having a melting point of 140°C or higher is preferably in a range of from 55.0% by mass to 89.9% by mass with respect to the total amount of a composition, and more preferably in a range of from 67.0% by mass to 88.0% by mass.

The content of an ethylene homopolymer is preferably from 1.0% by mass to 10.0% by mass with respect to the total amount of a composition, and more preferably from 2.0% by mass to 8.0% by mass.

A spunbond nonwoven fabric including such a composition has superior extensibility, high elongation at maximum load, and favorable flexibility, resistance to roll blocking, and heat-sealability.

The total content of a specific polypropylene and a specific polymer in the total mass of a composition is preferably 80% by mass or higher from the viewpoint of effective achievement of the object of the embodiment, more preferably 90% by mass or higher, and further preferably 95% by mass or higher.

(Propylene homopolymer having melting point of 140°C or higher)

A propylene homopolymer having a melting point of 140°C or higher includes a constituent unit derived from propylene, and has a melting point of 140°C or higher. The melting point is preferably 150°C or higher.

As a specific polypropylene, any crystalline resin that is produced or sold under the name of polypropylene, and is a resin having a melting point (Tm) of 140°C or higher, may be used. Examples of a commercial product may include a propylene homopolymer having a melting point of 155°C or higher, preferably in a range of from 157°C to 165°C.

Although there is no particular restriction on a melt flow rate (MFR: ASTMD-1238, 230°C, load 2160 g) with respect to a specific polypropylene insofar as melt spinning is possible, it is ordinarily in a range of from 1 g/10 min to 1000 g/10 min, preferably from 5 g/10 min to 500 g/10 min, and further preferably from 10 g/10 min to 100 g/10 min.

Only one kind of specific polypropylene may be used for a composition, or two or more kinds, having melting points, molecular weights, and crystal structures different from each other, may be used.

A preferable content of a specific polypropylene with respect to the total amount of a composition is as described above.

(Polyethylene)

The polyethylene is an ethylene homopolymer, such as a high pressure low density polyethylene, a linear low density polyethylene (so-called LLDPE), and a high density polyethylene (so-called HDPE).

As a polyethylene to be used for a composition, a high density polyethylene having a density in a range of from 0.941 g/cm$^3$ to 0.970 g/cm$^3$ is preferable among others from the viewpoint of improvement in extensibility, flexibility, and breaking strength.

Only one kind of a polyethylene may be used for a composition, or two or more kinds, having melting points, molecular weights, and crystal structures different from each other, may be used.

A preferable content of the polyethylene with respect to the total amount of a composition is as described above.

(Specific Polymer)

(Polymer shown in (I): a random copolymer of propylene and at least one $\alpha$-olefin selected from ethylene and a C4-C20 $\alpha$-olefin)

A polymer shown in (I) (hereinafter occasionally referred to as "polymer (I)") is a random copolymer including a constituent unit derived from propylene, and a constituent unit derived from at least one $\alpha$-olefin selected from ethylene and C4-C20 $\alpha$-olefins. Since a polymer (I) is a random copolymer, an obtained spunbond nonwoven fabric does not become sticky, and its flexibility may be improved, which is preferable.

There is no particular restriction on a polymer (I), insofar as it is a random copolymer including the constituent units.

Examples of a constituent unit copolymerizable with propylene include a constituent unit derived from ethylene, and a constituent unit derived from an $\alpha$-olefin selected from $\alpha$-olefins having a number of carbon atoms of 4 or higher, such as 1-butene, 1-hexene, 1-octene, and 4-methyl-1-pentene. Among others, a constituent unit derived from ethylene, and a constituent unit derived from an $\alpha$-olefin selected from $\alpha$-olefins having a number of carbon atoms of from 4 to 8 are preferable.

There may be only one kind of constituent unit derived from an $\alpha$-olefin in a polymer (I), or may be two or more kinds.

Specific and preferable examples of a polymer (I) include a propylene/1-butene random copolymer, a propylene/ethylene random copolymer, and a propylene/ethylene/1-butene random copolymer.

The total content of a constituent unit derived from propylene and a constituent unit derived from the $\alpha$-olefin other than propylene, such as ethylene, in the total constituent units contained in a polymer (I) is preferably 80 mol% or higher from the viewpoint of effective achievement of the object of the embodiment, more preferably 85 mol% or higher, and further preferably 90 mol% or higher.

A polymer (I) has preferably a crystallinity of 15% or less.

The melting point of a polymer (I) is defined as the peak top of a peak observed at the highest temperature side of a melting endothermic curve obtained using a differential scanning calorimeter (DSC) in which a polymer is kept in a nitrogen atmosphere at -40°C for 5 min and then the temperature is raised at 10°C/min. Specifically, using a differential scanning calorimeter (DSC-7, produced by PerkinElmer, Inc.), 5 mg of a sample is kept in a nitrogen atmosphere at -40°C for 5 min and then the temperature is raised at 10°C/min to obtain a melting endothermic curve, and the peak top of a peak observed at the highest temperature side of the curve is used as the melting point.

The melting point of a polymer (I) is 130°C or higher, and preferably 150°C or higher.

The crystallinity of a polymer (I) is calculated from a heat of fusion curve attributable to fusion of a main component in a melting endothermic curve obtained using a differential scanning calorimeter (DSC) in which a polymer is kept in a nitrogen atmosphere at -40°C for 5 min and then the temperature is raised at 10°C/min. Specifically, using a differential scanning calorimeter (DSC-7, produced by PerkinElmer, Inc.), 5 mg of a sample is kept in a nitrogen atmosphere at -40°C for 5 min and then the temperature is raised at 10°C/min to obtain a melting endothermic curve, and from a heat of fusion curve attributable to fusion of a main component in the melting endothermic curve, a crystallinity is calculated using the following Formula:

$$Crystallinity = \Delta H/\Delta H0 \times 100 \ (\%)$$

In the Formula, $\Delta H$ represents a heat quantity of fusion (J/g) determined from a heat of fusion curve attributable to fusion of a main component of an $\alpha$-olefin copolymer containing ethylene and propylene, and $\Delta H0$ represents the heat quantity of fusion (J/g) of the perfect crystal of the main component. Namely, when a main component is ethylene, $\Delta H0$ is 293 J/g; and when a main component is propylene, $\Delta H0$ is 210 J/g.

The crystallinity of a polymer (I) is more preferably 10% or less, and further preferably 8% or less.

The tensile modulus of a polymer (I) measured by a method according to JIS K 7161 (2011 version) is preferably 100 MPa or less, more preferably 40 MPa or less, and further preferably 25 MPa or less.

The melt flow rate (MFR, measurement conditions: 230°C, load 2160 g) of a polymer (I) measured by a method according to ASTM Standard D-1238 is in a range of from 30 g/10 min to 70 g/10 min from the viewpoint of securing favorable spinnability and superior stretching processing suitability.

Further, the ratio Mw/Mn (molecular weight distribution) of a weight-average molecular weight (Mw) to a number average molecular weight (Mn) of a polymer (I) is ordinarily from 1.5 to 5.0. From the viewpoint of availability of a conjugated fiber having more favorable spinnability and excellent fiber strength, the molecular weight distribution (Mw/Mn) of a polymer (I) is more preferably from 1.5 to 3.0. In this regard, favorable spinnability means that filament breakage does not occur during extrusion of a polymer (I) through a spinning nozzle and during stretching, and that fusion of filaments does not occur.

Mw and Mn of a polymer (I) may be measured by a method known in the art using GPC (gel permeation chromatography). The details of the measuring method will be described below.

(Polymer shown in (II): propylene homopolymer satisfying the following requirements (a) to (f))

A polymer shown in (II) (hereinafter occasionally referred to as "polymer (II)") is a polymer satisfying the following requirements (a) to (f).

(a)    [mmmm] = 20 mol% to 60 mol%:

When the meso pentad fraction [mmmm] of a polymer (II) is 20 mol% or higher, appearance of stickiness is suppressed, and when it is 60 mol% or less, the crystallinity does not become excessively high and therefore the elastic recovery may remain favorable. The meso pentad fraction [mmmm] is preferably from 30 mol% to 50 mol%, and more preferably from 40 mol% to 50 mol%.

A meso pentad fraction [mmmm], and a racemic pentad fraction [rrrr] and a racemic meso racemic meso pentad fraction [rmnn] described below are a meso fraction, a racemic fraction, and a racemic meso racemic meso fraction in a pentad unit in a polypropylene molecular chain to be measured from a signal of a methyl group in a $^{13}$C-NMR spectrum according to the method proposed by A. Zambelli, et al. "Macromolecules, 6, 925 (1973)". When a meso pentad fraction [mmmm] becomes high, the stereoregularity becomes also high. Triad fractions [mm], [rr], and [mr] described below are also calculated by the above method.

The $^{13}$C-NMR spectrum measurement may be conducted based on assignment of peaks proposed by A. Zambelli, et al. "Macromolecules, 8, 687 (1975)" using the following apparatus and conditions.

| | |
|---|---|
| Apparatus: | $^{13}$C-NMR apparatus, Model JNM-EX400, produced by JEOL Ltd. |
| Method: | Proton complete decoupling method |
| Concentration: | 220 mg/mL |
| Solvent: | 90:10 (volume ratio) mixture solvent of 1,2,4-trichlorobenzene and deuterated benzene |
| Temperature: | 130°C |
| Pulse width: | 45° |
| Pulse repetitiontime: | 4 sec |
| Integration: | 10000 times |

[Calculation Formula]

$$M = m/S \times 100$$

$$R = \gamma/S \times 100$$

$$S = P_{\beta\beta} + P_{\alpha\beta} + P_{\alpha\gamma}$$

| | | | |
|---|---|---|---|
| S: | The signal intensity of side chain methyl carbon atoms in total propylene units | | |
| $P_{\beta\beta}$: | 19.8 ppm to 22.5 ppm | | |
| $P_{\alpha\beta}$: | 18.0 ppm to 17.5 ppm | | |
| $P_{\alpha\gamma}$: | 17.5 ppm to 17.1 ppm | | |
| $\gamma$: | racemic pentad chain: | 20.7 ppm to 20.3 ppm | |
| m: | meso pentad chain: | 21.7 ppm to 22.5 ppm | |

$$(b) \qquad [rrrr]/(1\text{-}[mmmm]) \leq 0.1$$

The value of [rrrr]/[1-mmmm] is derived from the fractions of pentad units and is an index for showing the uniformity of tacticity distribution of constituent units derived from propylene in a polymer (II). When the value becomes high, it indicates a mixture of high tacticity polypropylene and atactic polypropylene, such as a conventional polypropylene produced with a conventional catalyst system, which causes stickiness.

When the [rrrr]/(1-[mmmm]) of a polymer (II) is 0.1 or less, the stickiness of a spunbond nonwoven fabric to be obtained may be suppressed. From this viewpoint, [rrrr]/(1-[mmmm]) is preferably 0.05 or less, and more preferably 0.04 or less.

$$(c) \qquad [rmrm] > 2.5 \text{ mol\%}$$

When the racemic meso racemic meso fraction [rmrm] of a polymer (II) is a value beyond 2.5 mol%, the randomness of the polymer (II) increases, and the elastic recovery of a spunbond nonwoven fabric is improved. [rmrm] is preferably 2.6 mol% or higher, and more preferably 2.7 mol% or higher. The upper limit is ordinarily approximately 10 mol%.

$$(d) \qquad [mm] \times [rr]/[mr]^2 \leq 2.0$$

$[mm] \times [rr]/[mr]^2$ is an index of the randomness of a polymer (II). When the value is 2.0 or less, an elastic nonwoven fabric acquires sufficient elastic recovery, and stickiness is suppressed. When $[mm] \times [rr]/[mr]^2$ approaches 0.25, the randomness becomes higher. From the viewpoint of securing sufficiently the elastic recovery, $[mm] \times [rr]/[mr]^2$ is preferably more than 0.25 and not more than 1.8, and more preferably from 0.5 to 1.5.

$$(e) \qquad \text{Weight-average molecular weight (Mw)} = 10{,}000 \text{ to } 200{,}000$$

When the weight-average molecular weight of a polymer (II), which is a propylene homopolymer, is 10,000 or more, the viscosity of the polymer (II) is not too low and appropriate, and consequently filament breakage during production of a spunbond nonwoven fabric from a composition may be suppressed. When the weight-average molecular weight is 200,000 or less, the viscosity of the polymer (II) is not too high, and the spinnability is improved. The weight-average molecular weight is preferably from 30,000 to 150,000, and more preferably from 50,000 to 150,000. A measurement method of a weight-average molecular weight of a polymer (II) will be described below.

$$(f) \qquad \text{Molecular weight distribution (Mw/Mn)} < 4$$

When molecular weight distribution (Mw/Mn) of a polymer (II) is less than 4, emergence of stickiness in an obtained spunbond nonwoven fabric is suppressed. The molecular weight distribution is preferably 3 or less.

The weight-average molecular weight (Mw) is a weight-average molecular weight in terms of polystyrene measured by a gel permeation chromatography (GPC) method with and under the following apparatus and conditions, and the molecular weight distribution (Mw/Mn) is a value calculated from a number average molecular weight (Mn) measured similarly and the weight-average molecular weight (Mw).

[GPC Measuring Apparatus]

| | |
|---|---|
| Column: | TOSO GMHHR-H (S) HT |
| Detector: | RI detector for liquid chromatogram, WATERS 150C |

[Measurement Conditions]

| | |
|---|---|
| Solvent: | 1,2,4-trichlorobenzene |
| Measurer ment temperature: | 145°C |
| Flow rate : | 1.0 mL/min |
| Sample c oncentration: | 2.2 mg/mL |
| Injected a amount: | 160 μL |
| Calibrati on curve: | Universal Calibration |
| Analysis program: | HT-GPC (Ver. 1.0) |

A polymer (II) preferably also satisfies the following requirement (g). (g) A melting point ($T_m$-D) defined as the peak top of a peak observed at the highest temperature side of a melting endothermic curve obtained using a differential scanning calorimeter (DSC) in which a polymer is kept in a nitrogen atmosphere at -10°C for 5 min and then the temperature is raised at 10°C/min, is from 0°C to 120°C.

When the melting point ($T_m$-D) of a polymer (II) is 0°C or higher, emergence of stickiness of a spunbond nonwoven fabric formed from a composition may be suppressed, and when it is 120°C or lower, sufficient elastic recovery may be obtained. From such viewpoints, the melting point ($T_m$-D) is more preferably from 0°C to 100°C, and further preferably from 30°C to 100°C.

In this regard, using a differential scanning calorimeter (DSC-7, produced by PerkinElmer, Inc.), in which 10 mg of a sample is kept in a nitrogen atmosphere at -10°C for 5 min and then the temperature is raised at 10°C/min, a melting endothermic curve is obtained, and the peak top of a peak observed at the highest temperature side of the curve may be used as the melting point ($T_m$-D).

The melt flow rate (MFR, measurement conditions: 230°C, load 2160 g) of a polymer (II) measured by a method according to ASTM Standard D-1238 is ordinarily, preferably in a range of from 1 g/10 min to 100 g/10 min, more preferably in a range of from 5 g/10 min to 100 g/10 min, and further preferably in a range of from 30 g/10 min to 70 g/10 min from the viewpoint of securing favorable spinnability and superior stretching processing suitability.

Further, the ratio Mw/Mn (molecular weight distribution) of a weight-average molecular weight (Mw) to a number average molecular weight (Mn) of a polymer (II) is ordinarily from 1.5 to 5.0. From the viewpoint of availability of a conjugated fiber having more favorable spinnability and excellent fiber strength, the molecular weight distribution (Mw/Mn) of a polymer (II) is more preferably from 1.5 to 3.0. In this regard, "to have favorable spinnability" means that filament breakage does not occur during extrusion of a polymer (II) through a spinning nozzle and during stretching, and that fusion of filaments does not occur.

Mw and Mn of a polymer (II) may be measured using GPC (gel permeation chromatography) by the previously described measuring method and measurement conditions.

A polymer (II) may be synthesized using a homogeneous catalyst called as a metallocene catalyst, for example, as described in WO2003/087172.

(Additive)

A composition may contain as some optional components various publicly known additives, such as an antioxidant, a heat-resistant stabilizer, a weathering stabilizer, an antistatic agent, a slip agent, an anti-fogging agent, a lubricant, a dye, a pigment, a natural oil, a synthetic oil, a wax, and a fatty acid amide, to the extent that the object of the embodiment is not impaired.

(Fatty Acid Amide)

A composition preferably contains a C15-C22 fatty acid amide. When a composition contains a fatty acid amide, a fatty acid amide is conceivably adsorbed on a fiber surface of a spunbond nonwoven fabric formed from the composition to modify a fiber surface, such that the flexibility, tactile feel, anti-blocking property, and the like are improved, and sticking of a nonwoven fabric fiber to a member of various rotating machines in an apparatus used in an embossing step or the like is suppressed more effectively.

Examples of a fatty acid amide having a number of carbon atoms of from 15 to 22 include a fatty acid monoamide compound, a fatty acid diamide compound, a saturated fatty acid monoamide compound, and an unsaturated fatty acid diamide compound.

In this regard, the number of carbon atoms of a fatty acid amide means herein the number of carbon atoms contained in a molecule, and the carbon atom in -CONH included in an amide is included in the number of carbon atoms. The number of carbon atoms of a fatty acid amide is more preferably from 18 to 22.

Specific examples of a fatty acid amide usable in a composition include palmitic acid amide (number of carbon atoms: 16), stearic acid amide (number of carbon atoms: 18), oleic acid amide (number of carbon atoms: 18), and erucic acid amide (number of carbon atoms: 22).

Fatty acid amides may be used singly or in a combination of two or more kinds in the composition.

The total content of a fatty acid amide with respect to the total amount of a composition is preferably in a range of from 0.1% by mass to 5.0% by mass from the viewpoint of effect.

A spunbond nonwoven fabric of the embodiment may be produced using one or two or more kinds of the compositions by an ordinary method.

As a general and exemplary method of producing a spunbond nonwoven fabric using the composition, there is a method in which a composition is melted in an extruder, the melted composition is melt spun using a spunbond nonwoven fabric forming machine with plural spinnerets, and continuous fibers formed by spinning are, after optional cooling, accumulated on a collection surface of the spunbond nonwoven fabric forming machine, followed by a heat-press treatment by embossing rolls.

There is no particular restriction on the melting temperature of a composition, insofar as it is not less than the softening temperature or melt temperature, and less than the thermal decomposition temperature of a composition used for spinning, and it may be decided appropriately according to physical properties of a composition used. Although the temperature of a spinneret depends on a composition used, considering physical properties of a propylene-containing polymer whose content in the composition is high, it is preferably set at a temperature of from 180°C to 240°C, more preferably from 190°C to 230°C, and further preferably from 200°C to 225°C.

There is no particular restriction on the temperature of cooling air for cooling a spun continuous fiber, insofar as it is a temperature at which a composition solidifies. In general, it is preferably from 5°C to 50°C, more preferably from 10°C to 40°C, and further preferably from 15°C to 30°C. In a case in which a spun fiber is stretched by air, the air velocity is ordinarily in a range of from 100 m/min to 10,000 m/min, and preferably from 500 m/min to 10,000 m/min.

Fibers of a spunbond nonwoven fabric may be partly heat-bonded. In this regard, the fibers may be pressed together using nip rolls before heat-bonding.

For a spunbond nonwoven fabric of the embodiment, a value obtained by dividing a 5% strength by the basis weight of the nonwoven fabric is preferably 0.2 N/25 mm/(g/m$^2$) or higher, and an accumulated value of stress integration values at 50% elongation is preferably 70 N/(g/m$^2$) or lower. Methods of obtaining a value obtained by dividing a 5% strength by the basis weight of a nonwoven fabric, and stress integration values at 50% elongation are described below.

A spunbond nonwoven fabric with the above preferable physical properties may be yielded using an open-system spunbond nonwoven fabric forming machine (hereinafter referred to as "extruder") described in detail below.

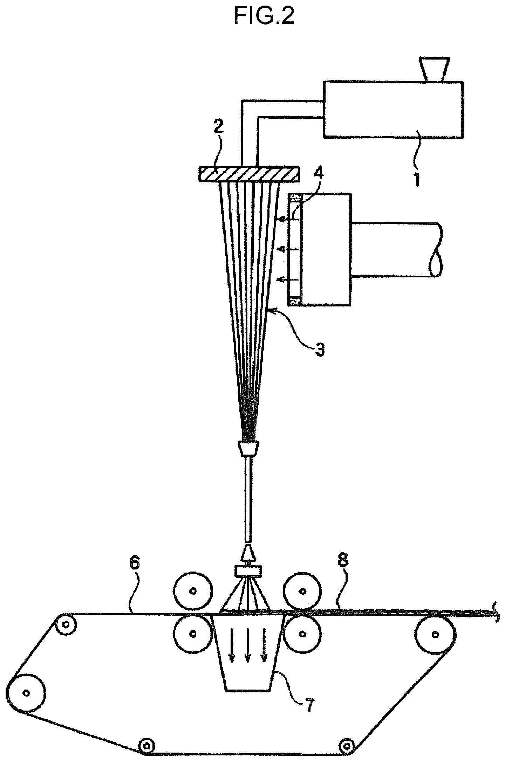

Fig. 2 is a schematic diagram of an open-system spunbond method of producing a nonwoven fabric in which a composition that is a raw material for a nonwoven fabric is used, and melt spun continuous fibers are stretched under cooling in the air.

An open-system spunbond method may include: a stretching step, in which a molten composition is extruded by an extruder 1 provided with a spinneret 2 through the spinneret 2 into the air to form continuous fibers 3, and the continuous fibers 3 are cooled, for example, by cooling air 4 in the air and stretched by an air sucker; a subsequent fiber distributing step, in which fibers are distributed when fibers collide, for example, with a distribution plate 6 oscillating in a non-machine direction of a line at a constant frequency; a subsequent conveying step, in which a nonwoven fabric accumulated on a belt is conveyed such that the basis weight is regulated constant; and an embossing step (not illustrated), in which embossing is conducted to develop strength as a sheet. The distribution plate 6 as a collector may be provided with a suction device 7 as shown in Fig. 2. Continuous fibers 3 arrive at a distribution plate 6 to form a spunbond nonwoven fabric 8.

[Nonwoven Fabric Laminate]

A spunbond nonwoven fabric of the embodiment may be used singly. Alternatively, a nonwoven fabric laminate may be formed corresponding to an intended use by laminating a spunbond nonwoven fabric of the embodiment and another layer. A nonwoven fabric laminate may include one or two or more layers other than a spunbond nonwoven fabric.

Specific examples of other layer include a knit fabric, a woven fabric, a nonwoven fabric other than a spunbond nonwoven fabric of the embodiment, and a film. There is no particular restriction on a method of layering (laminating) additionally another layer on a spunbond nonwoven fabric of the embodiment, and various methods including a heat bonding method, such as thermal embossing, and ultrasonic wave bonding; a mechanical entangling method, such as needle punching, and water-jetting; a method using an adhesive, such as a hot-melt adhesive, and a urethane resin adhesive; and extrusion lamination method, may be applicable.

Examples of other nonwoven fabric, which may be laminated with a spunbond nonwoven fabric of the embodiment to form a nonwoven fabric laminate, include various nonwoven fabrics known in the art, such as a spunbond nonwoven fabric other than a spunbond nonwoven fabric of the embodiment, a melt blown nonwoven fabric, a wet-laid nonwoven fabric, a dry-laid nonwoven fabric, an air-laid pulp nonwoven fabric, a flash-spun nonwoven fabric, and an opening nonwoven fabric. The nonwoven fabric may be a stretchable nonwoven fabric, or a non-stretchable nonwoven fabric. In this regard, a non-stretchable nonwoven fabric means one that does not generate recovery stress after stretching in the MD (travel direction of a nonwoven fabric, machine direction) or the CD (direction perpendicular to travel direction of a nonwoven fabric, cross-machine direction).

As a film to be laminated with a spunbond nonwoven fabric of the embodiment to form a nonwoven fabric laminate, for which air permeability is necessary, an air-permeable film or a moisture permeable film is preferable. Examples of an air-permeable film include various air permeable films known in the art including a film composed of a moisture permeable thermoplastic elastomer, such as a polyurethane elastomer, a polyester elastomer, and a polyamide elastomer; and a porous film, which is made porous by stretching a film composed of a thermoplastic resin containing inorganic fine particles or organic fine particles. As a thermoplastic resin used in a porous film, a polyolefin, such as a high pressure low density polyethylene, a linear low density polyethylene (so-called LLDPE), a high density polyethylene, a polypropylene, or a polypropylene random copolymer, or any combination thereof, is preferable.

In a case in which a nonwoven fabric laminate does not need air permeability, a film of a thermoplastic resin composed of one or more thermoplastic resins selected from polyethylene, polypropylene, and the like may be used.

Examples of a heat bonding method of heat-bonding a part of a nonwoven fabric laminate include various methods known in the art, such as a method using a means like ultrasonic waves, and a pre-bonding method using thermal embossing with embossing rolls, or using a hot air through. Among others, thermal embossing is preferable, because continuous fibers can be stretched efficiently in stretching.

In a case in which part of a nonwoven fabric laminate is heat-bonded by thermal embossing, the embossed area rate is ordinarily from 5% to 30%, and preferably from 5% to 20%, and the non-embossed unit area is 0.5 mm$^2$ or more, and preferably in a range of from 4 mm$^2$ to 40 mm$^2$.

A non-embossed unit area is the maximum area of a quadrangle inscribed in embossments in a minimum unit of a non-embossed part surrounded by embossed parts. Examples of an embossing shape include circle, ellipse, oval, square, rhombus, rectangle, quadrangle, and a continuous shape based on these shapes.

By stretching an obtained nonwoven fabric laminate, a stretchable nonwoven fabric laminate having stretchability may be formed.

There is no particular restriction on the method of stretching, and a method heretofore known in the art may be applied. The method of stretching may be a method of stretching partly, or a method of stretching entirely. Further, it may be a method of stretching uniaxially, or a method of stretching biaxially. Examples of a method of stretching in the machine direction (MD) include a method in which mixed fibers partly melt-bonded are passed through two or more sets of nip rolls. In this case, when the rotation speed of the nip rolls is increased in the order of product stream, a partly melt-bonded nonwoven fabric laminate can be stretched. Further, gear stretching processing may be conducted using a gear stretching device.

The stretching ratio is preferably 50% or higher, more preferably 100% or higher, and further preferably 200% or higher; and preferably 1000% or less, and more preferably 400% or less.

In the case of uniaxial stretching, either of the stretching ratio in the machine direction (MD) or that in the direction perpendicular thereto (CD) preferably satisfies the above stretching ratio. In the case of biaxial stretching, at least one of the stretching ratios in the machine direction (MD) and in the direction perpendicular thereto (CD) preferably satisfies the above stretching ratio.

By performing stretching processing at such a stretching ratio, an elastic continuous fiber in a spunbond nonwoven fabric is stretched, and a non-stretchable continuous fiber is plastically deformed and elongated corresponding to the stretching ratio. Similarly, also with respect to another layer laminated, an elastic layer is elastically deformed, and an inelastic layer is plastically deformed.

In a case in which an elastic layer and an inelastic layer are laminated and stretched in forming a nonwoven fabric laminate, when the stress is released, the elastic layer (continuous fibers included in the layer) is recovered elastically, while inelastic continuous fibers do not recover elastically but form folds to develop a bulky feel in a nonwoven fabric laminate. Since plastically deformed continuous fibers become thinner, the flexibility and tactile feel are improved, and an elongation limiting function may be imparted to a nonwoven fabric laminate

<Hygienic Material>

A hygienic material of the embodiment contains the spunbond nonwoven fabric of the embodiment.

A spunbond nonwoven fabric of the invention is superior in heat-sealability at a low temperature, and further has high extensibility and flexibility. Therefore, a spunbond nonwoven fabric of the embodiment is used favorably for a hygienic material.

In a production process for a hygienic material, such as a disposable diaper, a sanitary napkin, and a hygiene mask, nonwoven fabrics may be occasionally bonded together by heat-sealing. A spunbond nonwoven fabric of the embodiment has excellent heat-sealability. Therefore, even when a heat sealing time is shortened or a heat sealing temperature is lowered in a heat-sealing step for the purpose of increase in production speed, development of troubles, such as deterioration of bonding property, or hardening of a sealed part, may be inhibited. Consequently, when the spunbond nonwoven fabric of the embodiment is used, a hygienic material may be produced at a high productivity, while maintaining a soft texture, which is one of the advantages of a hygienic material of the embodiment.

Examples of a hygienic material include an absorbent article, such as a disposable diaper, and a sanitary napkin, a medical hygienic material, such as bandage, medical gauze, and towel, and a hygiene mask. A hygienic material, which may contain a spunbond nonwoven fabric of the embodiment, is not limited to the above, and is favorably applicable to any of hygienic material uses, for which extensibility and flexibility are required.

A hygienic material may contain a spunbond nonwoven fabric of the embodiment as a nonwoven fabric laminate containing a spunbond nonwoven fabric of the embodiment and another layer.

EXAMPLES

The embodiment of the present invention will now be described in more detail by way of Example thereof, provided that the invention is not limited to the Example, which is a mere embodiment of the invention.

Physical property values and the like in Examples and Comparative Examples are measured by the following methods.

(1) Basis Weight [g/m$^2$]

From a spunbond nonwoven fabric, 10 specimens in a size of 300 mm in the machine direction (MD) and 250 mm in the cross direction (CD) were sampled. The sampling sites were randomly determined 10 sites. Next, the mass (g) of each sampled specimen was measured using an electronic even balance (produced by Kensei Co., Ltd.). The mean value of the respective masses of the specimens was calculated. The obtained mean value was converted to mass (g) per 1 m$^2$, rounded off to a whole number, and defined as the basis weight [g/m$^2$] of a spunbond nonwoven fabric.

(2) Maximum Strength and Maximum Elongation [%]

In an isothermal chamber at a temperature of $20\pm2°C$ and a humidity of $65\pm2\%$ according to JIS Z 8703 (Standard Atmospheric Conditions for Testing), five specimens in a size of 25 cm in the machine direction (MD) and 5 cm in the cross direction (CD) were sampled from a spunbond nonwoven fabric according to JIS L 1906, 6.12.1 [A method]. The obtained specimens were subjected to a tensile test using a tensile tester (INSTRON 5564, produced by Instron Japan Company, Ltd.) under the conditions of chuck to chuck distance of 100 mm and tensile speed of 300 mm/min to measure a tensile load for five specimens. The mean value of the maximum measured values was defined as a maximum strength.

The elongation at the maximum strength was defined as a maximum elongation.

In addition to the maximum strength, a Strength Index expressed by the following Formula was also registered.

$$[\text{Formula}]: \quad \text{Strength Index} = [((\text{MD strength})^2 + (\text{CD strength})^2)/2]^{0.5}$$

(3) Value Obtained by Dividing MD 5% Strength by Basis Weight of Nonwoven Fabric

A tensile test in the machine direction (MD) was carried out according to the same protocol as in (2) maximum elongation. The mean value of tensile loads at the chuck to chuck distance of 105 mm (state elongated by 5% from 100 mm at the initial state of tensile test) with respect to five specimens was defined as a MD 5% strength. A value obtained by dividing MD 5% strength by basis weight of nonwoven fabric of the embodiment was calculated by dividing the obtained MD 5% strength by the basis weight, which is a weight per unit area of a nonwoven fabric.

(4) Accumulated Value of Stress Integration Values at 50% Elongation

In an isothermal chamber at a temperature of $20\pm2°C$ and a humidity of $65\pm2\%$ according to JIS Z 8703 (Standard Atmospheric Conditions for Testing), five nonwoven fabric specimens in a size of 60 mm in the machine direction (MD) and 50 mm in the cross direction (CD) were sampled according to JIS L 1906, 6.12.1 [A method]. The obtained specimens were subjected to a tensile test using a tensile tester (INSTRON 5564, produced by Instron Japan Company, Ltd.) under the conditions of chuck to chuck distance of 10 mm and tensile speed of 500 mm/min. With respect to the obtained tensile test curve, the displacement (abscissa) in terms of the chuck to chuck distance from 10 mm (initial state of tensile test) to 15 mm (state elongated by 50%) was equally divided into 60 segments, and the average value of the tensile load (ordinate) in each segment was calculated. The area (rectangle) defined by the increment of displacement (abscissa) and the tensile load (ordinate) in each segment was deemed as a value of stress integration in each segment. By summation of values of stress integration of 60 segments, an accumulated value of stress integration values at 50% elongation was obtained.

(5) Evaluation of Heat-Sealability

[Heat-Sealing Method]

From a spunbond nonwoven fabric, 10 specimens in a size of 100 mm in the machine direction (MD) and 100 mm in the cross direction (CD) were sampled. Next, two specimens were put together aligning the MD directions of the two and heat-sealed using a heat-seal tester (product name: HEAT SEAL TESTER, produced by Tester Sangyo Co., Ltd.) under the following conditions.

| | |
|---|---|
| Heat-seal bar width: | 10.0 mm |
| Heat-seal pressure: | 2.0 kg/cm$^2$ |
| Heat-seal time: | 1.0 sec |
| Heat-seal temperature: | Upper bar and lower bar were set at the same temperature (145°C, or 155°C) |
| Heat-seal direction: | Perpendicular to MD |

[Heat-Seal Strength (N/20 mm)]

A peel strength was measured for each of five specimens by conducting a peel test under the following conditions on a specimen heat-sealed under the above conditions using a constant speed tensile tester (product name: STROGRAPH, produced by Toyo Seiki Seisaku-Sho, Ltd.). The mean value of the peel strengths was deemed as a heat-seal strength.

| | |
|---|---|
| Specimen dimension: | width 20 mm, and length 50 mm |
| Tensile speed: | 30 mm/min |
| Ambient temperature during measurement: | 23°C |

"NG" in Table 1 and Table 2 stands for a situation in which the combined two specimens were not heat-sealed.

[Heat Resistance (against Burn) in Heat-Sealing]

A specimen heat-sealed under the above conditions was visually examined in terms of occurrence of burn, and rated according to the following criteria.

-Rating Criteria-

A: Burn has not occurred at a heat-sealed part.
B: Burn has occurred slightly at a heat-sealed part.
C: Obvious burn has occurred at a heat-sealed part.

[Shape Stability (against Contraction) in Heat-Sealing]

A specimen heat-sealed under the above conditions was visually examined in terms of occurrence of contraction, and rated according to the following criteria.

-Rating Criteria-

A: Contraction of a specimen by heat-sealing was not recognized.
B: Contraction of a specimen by heat-sealing was recognized.
C: Obvious contraction of a specimen by heat-sealing was recognized.

(6) Evaluation of Flexibility

[Flexibility (Touch)]

The touch of a spunbond nonwoven fabric was evaluated sensorily through a direct touch with hand, and rated according to the following criteria. A sensory evaluation was performed by 10 panelists, and the most voted rating was adopted.

-Rating criteria-

A: Touch was excellent, and flexibility was superior.
B: Touch was good, and flexibility was superior to the following C rating.
C: Touch was stiff and flexibility was inferior.

[Bending Resistance (Cantilever)]

The bending resistance [mm] of a spunbond nonwoven fabric was measured by conducting a cantilever test by the following method.
Specifically, the test was performed according to JIS L 1096, 8.19.1 [A method (45° cantilever method)].
From a spunbond nonwoven fabric, five specimens in a size of 2 cm×15 cm were cut out in each of the longitudinal direction (MD) and the transversal direction (CD).
An obtained specimen was placed on a horizontal platform which had a smooth surface and a 45° slope at an end, such that a short side of the specimen was set on the scale base line. Next, the specimen was slid gradually toward the slope by an appropriate method, and when the center point of an edge of the specimen touched the slope, the position of the other edge of the specimen was read on the scale.
A bending resistance is expressed by a traveled distance (mm) of a specimen. In the longitudinal direction (MD) and the transversal direction (CD), respectively, five specimens were measured, and the respective mean values were calculated, and a bending resistance was calculated by rounding off the value obtained according to the following Formula to one decimal place.

$$\text{Bending resistance} = \{[(\text{mean value of MD})^2 + (\text{mean value of CD})^2]/2\}^{1/2}$$

(7) Embossment Survival Rate [%]

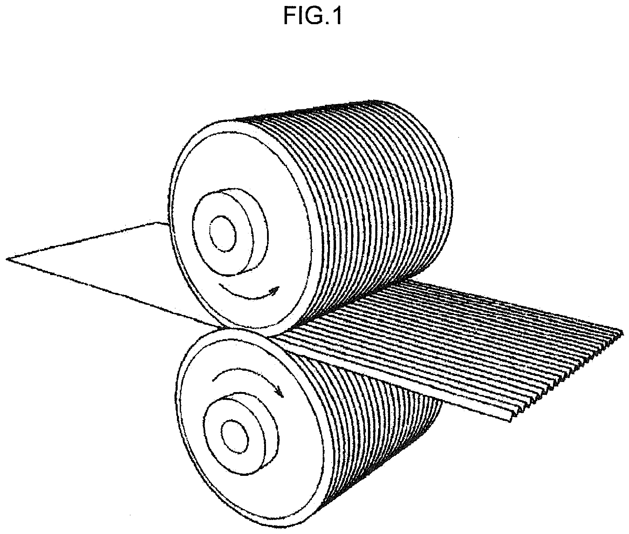

From a spunbond nonwoven fabric, a specimen in a size of 250 mm in the machine direction (MD) and 200 mm in the cross direction (CD) was sampled. The obtained specimen was inserted in a gear stretching device (gear processing machine) as shown in Fig. 1, such that the CD direction of the specimen coincides with the roll rotational direction. As the result, a spunbond nonwoven fabric gear-stretched in the MD direction (travelling direction of a nonwoven fabric) was obtained. With respect to respective gear rolls mounted on the gear processing machine, the diameters were 200 mm, the gear pitches were 2.5 mm, and the engagement depth of the two rolls was adjusted to 5.5 mm.

The embossment survival rate after gear stretching was evaluated by morphological observation with SEM on a spunbond nonwoven fabric, which was prepared by performing gear stretching on a spunbond nonwoven fabric obtained by a similar method to that used for the evaluation of touch. The higher embossment survival rate was rated as superior touch. The embossment survival rate was calculated using the following Formula.

$$\text{Embossment survival rate} = (\text{number of embossments not destructed} / \text{number of embossments observed}) \times 100$$

An "embossment not destructed" means an embossed part, where any of hole generation or fiber detachment in the embossed part, and fiber breakage at the embossed part and a boundary thereof were not recognized through SEM observation of the embossed part of a gear stretched spunbond nonwoven fabric.

From the fact that the survival rate of embossments formed by stretching processing using a gear processing machine is excellent, and fiber breakage of a spunbond nonwoven fabric in an embossed part or at the boundary thereof, or breakage of a nonwoven fabric caused by the fiber breakage does not occur during stretching processing, it can be confirmed that the stretching processing suitability of a spunbond nonwoven fabric is superior.

[Example 1]

<Production of Spunbond Nonwoven Fabric>

A mixture of 71.7% by mass of a propylene homopolymer (1) having a MFR of 60 g/10 min (measured according to ASTM D1238 at a temperature of 230°C, and a load of 2.16 kg), a density of 0.91 g/cm$^3$, and a melting point of 160°C; 20% by mass of a propylene random copolymer (2: polymer (I)) (copolymer of propylene and ethylene, polymerization molar ratio 97:3) having a MFR of 60 g/10 min (measured according to ASTM D1238 at a temperature of 230°C, and a load of 2.16 kg), a density of 0.91 g/cm$^3$, and a melting point of 142°C; 8% by mass of a high density polyethylene (3: hereinafter referred to as "polyethylene") having a MFR of 5 g/10 min (measured according to ASTM D1238 at a temperature of 190°C, and a load of 2.16 kg), a density of 0.95 g/cm$^3$, and a melting point of 134°C; and 0.3% by mass of erucic acid amide was melted using a 75 mm$\phi$ extruder, and melt spun using a spunbond nonwoven fabric forming machine having a 2557-hole spinneret (length in a direction perpendicular to the machine direction on a collection surface: 800 mm) under conditions that both the resin temperature and the die temperature were 220°C, the cooling air temperature was 20°C, and the stretching air velocity was 5233 m/min according to a spunbond method. The fibers were accumulated on a collection surface, and subjected to a heating and pressing treatment with embossing rolls (embossed area rate (heat-press bonding rate): 18%, embossing temperature: 100°C) to produce a spunbond nonwoven fabric having a total basis weight of 18.0 g/m$^2$.

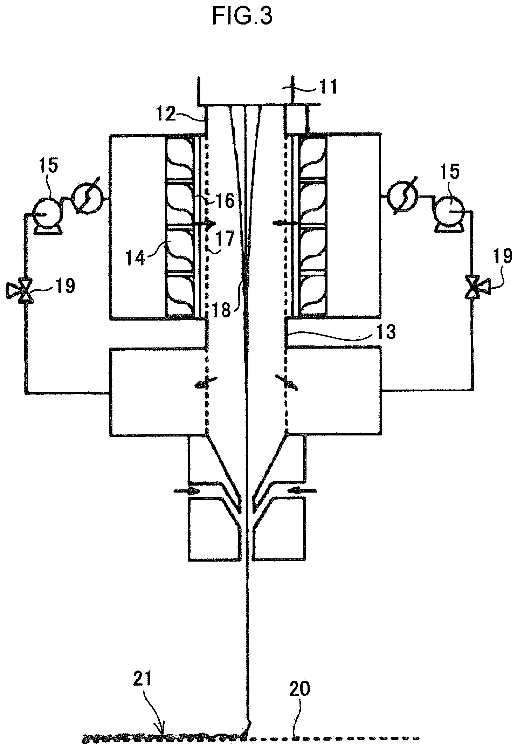

In this regard, in Examples 1 to 3, spunbond nonwoven fabrics were produced through a closed system spinning process shown in Fig. 3 (hereinafter referred to as "closed system spunbond method").

Fig. 3 is a schematic diagram of a closed system spunbond method. In the closed system spunbond method, a composition extruded through a spinneret 11 passes through a neck 12 of the closed system spunbond forming apparatus, and is cooled in a hermetically closed cooling chamber 13 to form continuous fibers 18, which arrive at a capturing device 20 to form a spunbond nonwoven fabric 21. In a cooling chamber 13, a blower 15 provided with a looper 14 supplies cooling air into the cooling chamber 13. The supply amount of cooling air into the cooling chamber 13 is adjusted by a blower 15, a switching valve 19 for regulating cooling air to be sent to the blower 15, and opening or closing of a damper 16.

The obtained spunbond nonwoven fabric of Example 1 was evaluated by the aforedescribed evaluation methods. The results are shown in the following Table 1.

The obtained spunbond nonwoven fabric of Example 1 had high extensibility and excellent touch, and was superior in flexibility.

Further, as shown in the following Table 1, the spunbond nonwoven fabric was superior in heat resistance in heat-sealing such that burn did not occur at a heat-sealed part. Further, contraction of a specimen due to het-sealing was minimal to demonstrate superior shape stability.

Further, as the result of evaluation of embossment survival rate, the embossment survival rate was high to demonstrate superior stretching processing suitability.

[Example 2]

<Synthesis of Low Crystallinity Polypropylene>

Into a 0.2 m$^3$-capacity reactor made of a stainless steel provided with a stirrer, n-heptane is continuously supplied at 20 L/h, and also triisobutylaluminum at 15 mmol/h, and a catalyst component obtained by contacting in advance dimethylanilinium tetrakis(pentafluorophenyl) borate, (1,2'-dimethylsilylene)(2,1'-dimethylsilylene)-bis(3-trimethylsilylmethylindenyl) zirconium dichloride, triisobutylaluminum, and propylene at 6 μmol/h in terms of zirconium.

Propylene and hydrogen were continuously supplied such that at a polymerization temperature of 70°C, a hydrogen concentration in a gas phase was kept at 8 mol%, and the total pressure in the reactor was kept at 0.7 MPa·G.

SUMILIZER GP (produced by Sumitomo Chemical Co., Ltd.) was added to the obtained polymerization solution to 1000 ppm, and by removing the solvent, a propylene polymer was obtained.

The weight-average molecular weight (Mw) of the obtained propylene polymer was $1.2 \times 10^4$, and the Mw/Mn was 2. The [mmmm] obtained from a NMR analysis was 46 mol%, [rrrr]/(1-[mmmm]) was 0.038, [rmrm] was 2.7 mol%, and [mm]×[rr]/[mr]$^2$ was 1.5.

The low crystallinity polypropylene (polymer (II)) obtained as above was hereinafter referred to as "LMPP1".

<Production of Spunbond Nonwoven Fabric>

A spunbond nonwoven fabric of Example 2 was obtained by the same method as Example 1 except that a mixture of 71.7% by mass of a propylene homopolymer (1) having a MFR of 60 g/10 min (measured according to ASTM D1238 at a temperature of 230°C, and a load of 2.16 kg), a density of 0.91 g/cm$^3$, and a melting point of 160°C; 20% by mass of LMPP1 (2) having a MFR of 50 g/10 min (measured according to ASTM D1238 at a temperature of 230°C, and a load of 2.16 kg), and a melting point of 75°C; 8% by mass of a high density polyethylene (3) having a MFR of 5 g/10 min (measured according to ASTM D1238 at a temperature of 190°C, and a load of 2.16 kg), a density of 0.95 g/cm$^3$, and a melting point of 134°C; and 0.3% by mass of erucic acid amide was used, and a heating and pressing treatment was conducted with embossing rolls (embossed area rate (heat-press bonding rate): 18%, embossing temperature: 95°C).

The obtained spunbond nonwoven fabric of Example 2 was evaluated by the aforedescribed evaluation methods. The results are shown in the following Table 1.

As the result of evaluation, the spunbond nonwoven fabric of Example 2 had high extensibility and excellent touch, and was superior in flexibility.

Further, as the result of measurement of the heat-seal strength, the heat resistance in heat-sealing was superior such that burn did not occur at a heat-sealed part. Further, contraction of a specimen due to het-sealing was minimal to demonstrate superior shape stability.

Further, the embossment survival rate of the spunbond nonwoven fabric was high to demonstrate superior stretching processing suitability.

[Example 3]

<Synthesis of Propylene/ethylene/1-butene Copolymer>

Into a 2000 mL-capacity polymerization apparatus thoroughly purged with nitrogen, 833 mL of dry hexane, 100 g of 1-butene, and 1.0 mmol of triisobutylaluminum were charged at normal temperature. Then the internal temperature of the polymerization apparatus was elevated to 40°C, and propylene is supplied to raise the pressure. Thereafter, ethylene was supplied to adjust the internal pressure to 0.8 MPa.

Then, a toluene solution containing 0.001 mmol of dimethylmethylene-(3-tert-butyl-5-methylcyclopentadienyl) fluorenyl zirconium dichloride, and 0.3 mmol (in terms of aluminum) of methylaluminoxane (produced by Tosoh Finechem Corporation) was added into the polymerization vessel. Then, polymerization was continued for 20 min, while maintaining the internal temperature at 40°C and the internal pressure at 0.8 MPa by feeding ethylene. Thereafter, the polymerization was terminated by adding 20 mL of methanol. After depressure, a polymer was precipitated from the polymerization solution in 2 L of methanol, followed by drying in vacuum at 130°C for 12 hours.

The propylene content in the obtained polymer was 78 mol%, the ethylene content was 16 mol%, and the 1-butene content was 6 mol%. The melting point was 161°C, the crystallinity was 6%, and the tensile modulus was 23.5 MPa.

The thus obtained propylene/ethylene/1-butene (C2/C3/C4) copolymer (polymer (I)) is hereinafter referred to as "PEB-1".

<Production of Spunbond Nonwoven Fabric>

A spunbond nonwoven fabric of Example 3 was obtained by the same method as Example 1 except that a mixture of 71.7% by mass of a propylene homopolymer (1) having a MFR of 60 g/10 min (measured according to ASTM D1238 at a temperature of 230°C, and a load of 2.16 kg), a density of 0.91 g/cm$^3$, and a melting point of 160°C; 20% by mass of PEB-1 (2) having a MFR of 30 g /10 min (measured according to ASTM D1238 at a temperature of 230°C, and a load of 2.16 kg); 8% by mass of a high density polyethylene (3) having a MFR of 5 g/10 min (measured according to ASTM D1238 at a temperature of 190°C, and a load of 2.16 kg), a density of 0.95 g/cm$^3$, and a melting point of 134°C; and 0.3% by mass of erucic acid amide was used, and a heating and pressing treatment was conducted with embossing rolls (embossed area rate (heat-press bonding rate): 18%, embossing temperature: 95°C).

The obtained spunbond nonwoven fabric of Example 3 was evaluated by the aforedescribed evaluation methods. The results are shown in the following Table 1.

The spunbond nonwoven fabric of Example 3 had high extensibility and excellent touch, and was superior in flexibility.

Further, as the result of measurement of the heat-seal strength, the spunbond nonwoven fabric had such superior heat resistance in heat-sealing that burn did not occur at a heat-sealed part. Further, contraction of a specimen due to het-sealing was minimal to demonstrate superior shape stability.

Further, as the result of evaluation of the embossment survival rate, the embossment survival rate of the spunbond nonwoven fabric was high to demonstrate superior stretching processing suitability.

[Example 4]

<Production of Spunbond Nonwoven Fabric>

A spunbond nonwoven fabric was obtained similarly to Example 2 except that a mixture of 87.2% by mass of a propylene homopolymer (1) having a MFR of 60 g/10 min (measured according to ASTM D1238 at a temperature of 230°C, and a load of 2.16 kg), a density of 0.91 g/cm$^3$, and a melting point of 160°C; 10% by mass of LMPP1 (2) having a MFR of 50 g/10 min (measured according to ASTM D1238 at a temperature of 230°C, and a load of 2.16 kg), and a melting point of 75°C; 2.5% by mass of a high density polyethylene (3) having a MFR of 5 g/10 min (measured according to ASTM D1238 at a temperature of 190°C, and a load of 2.16 kg), a density of 0.95 g/cm$^3$, and a melting point of 134°C; and 0.3% by mass of erucic acid amide was used by an open-system spunbond method with an open-system spunbond nonwoven fabric forming machine 1 shown in Fig. 2, and a heating and pressing treatment was conducted with embossing rolls (embossed area rate (heat-press bonding rate): 18%, embossing temperature: 95°C).

The evaluation results are shown in the following Table 1. The value obtained by dividing 5% strength of the obtained spunbond nonwoven fabric of Examples 4 by the basis weight of the nonwoven fabric was 0.26 N/25 mm/(g/m$^2$), and the accumulated value of stress integration values at 50% elongation was 53 N/(g/m$^2$).

[Comparative Example 1]

<Production of Spunbond Nonwoven Fabric>

A spunbond nonwoven fabric of Comparative Example 1 was obtained by the same method as Example 1 except that a mixture of 91.7% by mass of a propylene homopolymer (1) having a MFR of 60 g/10 min (measured according to ASTM D1238 at a temperature of 230°C, and a load of 2.16 kg), a density of 0.91 g/cm$^3$, and a melting point of 160°C; 8% by mass of a high density polyethylene (3) having a MFR of 5 g/10 min (measured according to ASTM D1238 at a temperature of 190°C, and a load of 2.16 kg), a density of 0.95 g/cm$^3$, and a melting point of 134°C; and 0.3% by mass of erucic acid amide was used, and a heating and pressing treatment was conducted with embossing rolls (embossed area rate (heat-press bonding rate): 18%, embossing temperature: 105°C).

The obtained spunbond nonwoven fabric of Comparative Example 1 was evaluated by the aforedescribed evaluation methods. The results are shown in the following Table 1.

A spunbond nonwoven fabric of Comparative Example 1 yielded from a composition not containing a specific polymer exhibited high extensibility, however its touch was unsatisfactory, and the flexibility was inferior.

As the result of measurement of the heat-seal strength, the heat-seal strength at 145°C of the spunbond nonwoven fabric was low and insufficient compared to respective nonwoven fabrics of Examples.

As the result of evaluation of the embossment survival rate, the spunbond nonwoven fabric exhibited a low embossment survival rate, and inferior stretching processing suitability.

The value obtained by dividing 5% strength of the obtained spunbond nonwoven fabric of Comparative Example 1 by the basis weight of the nonwoven fabric was 0.17 N/25 mm/(g/m$^2$), which was a value inadequate for practical use.

[Comparative Example 2]

<Production of Spunbond Nonwoven Fabric>

A spunbond nonwoven fabric of Comparative Example 2 was obtained by the same method as Example 1 except that a mixture of 99.7% by mass of a propylene homopolymer (1) having a MFR of 60 g/10 min (measured according to ASTM D1238 at a temperature of 230°C, and a load of 2.16 kg), a density of 0.91 g/cm$^3$, and a melting point of 160°C; and 0.3% by mass of erucic acid amide was used, and a heating and pressing treatment was conducted with embossing rolls (embossed area rate (heat-press bonding rate): 18%, embossing temperature: 135°C).

The obtained spunbond nonwoven fabric of Comparative Example 2 was evaluated by the aforedescribed evaluation methods. The results are shown in the following Table 1.

A spunbond nonwoven fabric of Comparative Example 2 yielded from a composition not containing a specific polymer and a polyethylene exhibited insufficient extensibility, and also its touch was unsatisfactory, and the flexibility was inferior.

As the result of measurement of the heat-seal strength, the spunbond nonwoven fabric was not bonded by heat-sealing at both the heat-sealing temperatures of 145°C and 155°C.

As the result of evaluation of the embossment survival rate, the spunbond nonwoven fabric was inferior in stretching processing suitability, such that breakage occurred in gear stretching, due to insufficient extensibility.

[Comparative Example 3]

<Production of Spunbond Nonwoven Fabric>

A spunbond nonwoven fabric of Comparative Example 3 was obtained by the same method as Example 1 except that a mixture of 79.7% by mass of a propylene homopolymer (1) having a MFR of 60 g/10 min (measured according to ASTM D1238 at a temperature of 230°C, and a load of 2.16 kg), a density of 0.91 g/cm$^3$, and a melting point of 160°C; 20% by mass of a propylene random copolymer (2) (copolymer of propylene and ethylene, polymerization molar ratio 97:3) having a MFR of 60 g/10 min (measured according to ASTM D1238 at a temperature of 230°C, and a load of 2.16 kg), a density of 0.91 g/cm$^3$, and a melting point of 142°C; and 0.3% by mass of erucic acid amide was used, and a heating and pressing treatment was conducted with embossing rolls (embossed area rate (heat-press bonding rate): 18%, embossing temperature: 135°C).

The obtained spunbond nonwoven fabric of Comparative Example 3 was evaluated by the aforedescribed evaluation methods. The results are shown in the following Table 1.

A spunbond nonwoven fabric of Comparative Example 3 yielded from a composition not containing a polyethylene exhibited insufficient extensibility, and also its touch was unsatisfactory, and the flexibility was inferior.

As the result of measurement of the heat-seal strength, the spunbond nonwoven fabric was not bonded by heat-sealing at both the heat-sealing temperatures of 145°C and 155°C.

As the result of evaluation of the embossment survival rate, the spunbond nonwoven fabric was inferior in stretching processing suitability, such that breakage occurred in gear stretching, due to insufficient extensibility.

[Comparative Example 4]

<Production of Spunbond Nonwoven Fabric>

A spunbond nonwoven fabric of Comparative Example 4 was obtained by the same method as Example 1 except that a mixture of 79.7% by mass of a propylene homopolymer (1) having a MFR of 60 g/10 min (measured according to ASTM D1238 at a temperature of 230°C, and a load of 2.16 kg), a density of 0.91 g/cm$^3$, and a melting point of 160°C; 20% by mass of LMPP1 (2); and 0.3% by mass of erucic acid amide was used, and a heating and pressing treatment was conducted with embossing rolls (embossed area rate (heat-press bonding rate): 18%, embossing temperature: 135°C).

The obtained spunbond nonwoven fabric of Comparative Example 4 was evaluated by the aforedescribed evaluation methods. The results are shown in the following Table 1.

A spunbond nonwoven fabric of Comparative Example 4 yielded from a composition not containing a polyethylene exhibited insufficient extensibility, and also its touch was insufficient, and the flexibility was inferior.

As the result of measurement of the heat-seal strength, the spunbond nonwoven fabric was not bonded by heat-sealing at both the heat-sealing temperatures of 145°C and 155°C.

As the result of evaluation of the embossment survival rate, the spunbond nonwoven fabric was inferior in stretching processing suitability, such that breakage occurred in gear stretching, due to insufficient extensibility.

[Comparative Example 5]

<Production of Spunbond Nonwoven Fabric>

A spunbond nonwoven fabric of Comparative Example 5 was obtained by the same method as Example 1 except that a mixture of 79.7% by mass of a propylene homopolymer (1) having a MFR of 60 g/10 min (measured according to ASTM D1238 at a temperature of 230°C, and a load of 2.16 kg), a density of 0.91 g/cm$^3$, and a melting point of 160°C; 20% by mass of a block copolymer described in Japanese Patent No. 3798018 (copolymer of 76% of isotactic polypropylene, and 20% of propylene: MOTENLL KS057P; hereinafter referred to as "block copolymer"); and 0.3% by mass of erucic acid amide was used, and a heating and pressing treatment was conducted with embossing rolls (embossed area rate (heat-press bonding rate): 18%, embossing temperature: 135°C).

The obtained spunbond nonwoven fabric of Comparative Example 5 was evaluated by the aforedescribed evaluation methods. The results are shown in the following Table 1.

A spunbond nonwoven fabric of Comparative Example 5 using the block copolymer exhibited sufficient extensibility, however it had severe stickiness, unsatisfactory touch, and inferior flexibility.

As the result of evaluation of the embossment survival rate, the spunbond nonwoven fabric was inferior in stretching processing suitability, such that breakage occurred in gear stretching.

The description of "-" in the following Table 1 and Table 2 means that the relevant evaluation was not conducted.

TABLE 1

| | | | | Example 1 | Example 2 | Example 3 | Example 4 |
|---|---|---|---|---|---|---|---|
| Production process (open system or closed system spunbond method) | | | | Closed system | Closed system | Closed system | Open system |
| Composition of raw material (% by mass) | Components (% by mass) | Propylene homopolymer with melting point of 140°C or higher | Polymer (I) | 71.7 | 71.7 | 71.7 | 87.2 |
| | | Propylene random copolymer | Polymer (I) | 20 | 0 | 0 | 0 |
| | | LMPP1 | Polymer (II) | 0 | 20 | 0 | 10 |
| | | PEB-1 | Polymer (I) | 0 | 0 | 20 | 0 |
| | | Polyethylene | | 8 | 8 | 8 | 2.5 |
| | | Block copolymer | | 0 | 0 | 0 | 0 |
| | | Erucic acid amide | Additive | 0.3 | 0.3 | 0.3 | 0.3 |
| Production condition | Embossing temperature | | [°C] | 100 | 95 | 95 | 95 |
| | Basis weight | | [g/m²] | 18 | 18 | 18 | 18 |

**20**

(continued)

| | | | | Example 1 | Example 2 | Example 3 | Example 4 |
|---|---|---|---|---|---|---|---|
| Production process (open system or closed system spunbond method) | | | | Closed system | Closed system | Closed system | Open system |
| Performance evaluation | Maximum strength | MD | [N/50 mm] | 31 | 25 | 38 | 44 |
| | | CD | [N/50 mm] | 12 | 9 | 14 | 17 |
| | | INDEX | [N/50 mm] | 0.92 | 0.74 | 1.13 | 1.31 |
| | Maximum elongation | MD | [%] | 169 | 152 | 165 | 70 |
| | | CD | [%] | 151 | 135 | 147 | 62 |
| | Cantilever | MD | [mm] | 36 | 28 | 26 | - |
| | | CD | [mm] | 20 | 16 | 18 | - |
| | | AVE | [mm] | 29 | 23 | 22 | - |
| | Flexibility | Sensory evaluation | | A | A | A | A |
| | Heat-sealability | 145°C | Strength | [N/20 mm] | 0.07 | 0.10 | 0.07 | - |
| | | | Burn | [-] | A | A | A | - |
| | | | Contraction | [-] | A | A | A | - |
| | | 155°C | Strength | [N/20 mm] | 1.44 | 1.52 | 0.84 | - |
| | | | Burn | [-] | A | A | A | - |
| | | | Contraction | [-] | A | A | A | - |
| | Embossment survival rate | | [%] | 57% | 93% | 53% | 43% |

(continued)

| | | | | Example 1 | Example 2 | Example 3 | Example 4 |
|---|---|---|---|---|---|---|---|
| Production process (open system or closed system spunbond method) | | | | Closed system | Closed system | Closed system | Open system |
| processing) | Maximum strength (after stretching processing) | MD | [N/50 mm/(g/m$^2$)] | 1.28 | 1.53 | 1.07 | 0.97 |
| | Accumulated value of stress integration values at 50% elongation | | [N/25 mm/(g/m$^2$)] | - | - | - | 53 |
| | Value obtained by dividing MD 5% strength by basis weight of nonwoven fabric | MD | [N/25 mm/(g/m$^2$)] | - | - | - | 0.26 |

TABLE 2

| Composition of raw material (% by mass) | Components (% by mass) | Propylene homopolymer with melting point of 140°C or higher | | Comparative Example 1 | Comparative Example 2 | Comparative Example 3 | Comparative Example 4 | Comparative Example 5 |
|---|---|---|---|---|---|---|---|---|
| Production process (open system or closed system spunbond method) | | | | Closed system | Closed system | Closed system | Closed system | Closed system |
| Composition of raw material (% by mass) | Components (% by mass) | Propylene homopolymer with melting point of 140°C or higher | | 91.7 | 99.7 | 79.7 | 79.7 | 79.7 |
| | | Propylene random copolymer | Polymer (I) | 0 | 0 | 20 | 0 | 0 |
| | | LMPP1 | Polymer (II) | 0 | 0 | 0 | 20 | 0 |
| | | PEB-1 | Polymer (I) | 0 | 0 | 0 | 0 | 0 |
| | | Polyethylene | | 8 | 0 | 0 | 0 | 0 |
| | | Block copolymer | | 0 | 0 | 0 | 0 | 20 |
| | | Erucic acid amide | Additive | 0.3 | 0.3 | 0.3 | 0.3 | 0.3 |
| Production condition | Embossing temperature | | [°C] | 105 | 135 | 135 | 135 | 135 |
| | Basis weight | AVE | [g/m$^2$] | 18 | 18 | 18 | 18 | 18 |

(continued)

| | | | | Comparative Example 1 | Comparative Example 2 | Comparative Example 3 | Comparative Example 4 | Comparative Example 5 |
|---|---|---|---|---|---|---|---|---|
| Performance evaluation | Maximum strength | MD | [N/50 mm] | 35 | 44 | 43 | 44 | 14 |
| | | CD | [N/50 mm] | 13 | 12 | 11 | 9 | 6 |
| | | INDEX | [N/50 mm] | 1.04 | 1.27 | 1.23 | 1.24 | 0.42 |
| | Maximum elongation | MD | [%] | 160 | 36 | 40 | 38 | 162 |
| | | CD | [%] | 143 | 28 | 39 | 33 | 144 |
| | Cantilever | MD | [mm] | 42 | 62 | 58 | 51 | 35 |
| | | CD | [mm] | 20 | 33 | 30 | 32 | 28 |
| | | AVE | [mm] | 33 | 69 | 74 | 74 | 32 |
| | Flexibility | sensory evaluation | | C | c | B | A | C |
| | Heat-sealability | 145°C | Strength | [N/20 mm] | 0.04 | NG | NG | NG | NG |
| | | | Burn | [-] | A | A | A | A | A |
| | | | Contraction | [-] | A | A | A | A | A |
| | | 155°C | Strength | [N/20 mm] | 0.26 | NG | NG | NG | NG |
| | | | Burn | [-] | A | A | A | A | A |
| | | | Contraction | [-] | A | A | A | A | A |
| | Embossment survival rate | | [%] | 33% | breakage | breakage | breakage | 18% |

EP 3 321 408 B1

(continued)

| | | | Comparative Example 1 | Comparative Example 2 | Comparative Example 3 | Comparative Example 4 | Comparative Example 5 |
|---|---|---|---|---|---|---|---|
| Maximum strength (after stretching processing) | MD | [N/50 mm/(g/m²)] | 0.78 | unevaluable | unevaluable | unevaluable | 0.39 |
| Accumulated value of stress integration values at 50% elongation | | [N/25 mm/(g/m²)] | 39 | - | - | - | - |
| Value obtained by dividing MD 5% strength by basis weight of nonwoven fabric | MD | [N/25 mm/(g/m²)] | 0.17 | - | - | - | - |

From the results in Table 1 and Table 2, spunbond nonwoven fabrics of the embodiment obtained in Example 1 to Example 4 had a high embossment survival rate after gear stretching, and experienced no breakage in gear stretching, and all of them were understandably superior to spunbond nonwoven fabrics of Comparative Examples in terms of stretching processing suitability.

As obvious from Table 1, since all of spunbond nonwoven fabrics of Example 1 to Example 4 were superior in heat-sealability, and superior in embossment survival rate as described above, it is inferable that they have superior stretching processing suitability. Further, all of spunbond nonwoven fabrics of Example 1 to Example 4 were superior in extensibility and flexibility.

Therefore, all of spunbond nonwoven fabrics of Examples had superior processing suitability. From the evaluation results, it is clear that a spunbond nonwoven fabric of the embodiment is suitable for a use as a hygienic material requiring extensibility, flexibility, and processing suitability.

## Claims

1. A fabric which is a spunbond nonwoven fabric, consisting of a composition comprising

   (1) a propylene homopolymer having a melting point of $\geq$ 140°C,
   (2) an ethylene homopolymer having a density of 0.941-0.970 g/cm$^3$, and
   (3) at least one polymer selected from (I) and (II):

   (I) a random copolymer that includes a constituent unit derived from propylene and at least one constituent unit selected from a constituent unit derived from ethylene and a constituent unit derived from a $C_{4-20}$-$\alpha$-olefin, that has a melting point of $\geq$ 130°C, and that has a melt flow rate of 30-70 g/10 min, whereby the melting point is defined as the peak top of a peak observed at the highest temperature side of a melting endothermic curve obtained using a differential scanning calorimeter (DSC) in which a polymer is kept in a nitrogen atmosphere at -40 °C for 5 min and then the temperature is raised at 10°C/min and the melt flow rate is measured by a method according to ASTM Standard D-1238 at 230°C, load 2160 g and
   (II) a propylene homopolymer having a melting point of < 120 °C whereby the melting point is defined as the peak top of a peak observed at the highest temperature side of a melting endothermic curve obtained using a differential scanning calorimeter (DSC) in which a polymer is kept in a nitrogen atmosphere at -10°C for 5 min and then the temperature is raised at 10°C/min, and satisfying the following (a)-(f):

   (a) [mmmm] = 20-60 mol-%
   (b) [rrrr]/(1-[mmmm]) $\leq$ 0.1
   (c) [rmrm] > 2.5 mol%
   (d) [mm]$\times$[rr]/[mr]$^2$ $\leq$ 2.0
   (e) weight-average molecular weight (Mw) = 10,000-200,000, whereby the weight-average molecular weight is measured according to the method of the description
   (f) molecular weight distribution (Mw/Mn) < 4, whereby the molecular weight distribution is determined according to the method of the description,

   wherein [mmmm] is a meso pentad fraction, [rrrr] is a racemic pentad fraction, [rmrm] is a racemic meso racemic meso pentad fraction, and [mm], [rr] and [mr] each are a triad fraction measured according to the method of the description, and wherein the total content of the polymer selected from (I) and (II), based on the total amount of the composition, is 5-30 mass-%.

2. The fabric of claim 1, wherein the composition contains, based on the total amount of the composition, 0.1-5.0 mass-% of a $C_{15-22}$-fatty acid amide.

3. The fabric of claim 1 or 2, wherein the composition contains, based on the total amount of the composition, 55.0-89.9 mass-% of the propylene homopolymer (1).

4. The fabric of any of claims 1-3, wherein the composition contains, based on the total amount of the composition, 1.0-10.0 mass-% of the ethylene homopolymer (2).

5. The fabric of any of claims 1-4, wherein the polymer (I) is a random copolymer of propylene and ethylene.

6. The fabric of any of claims 1-5, wherein the polymer (I) is a random copolymer of propylene, ethylene, and 1-butene.

7. The fabric of any of claims 1-6, wherein a value obtained by dividing the 5% strength of the fabric by the basis weight thereof is $\geq$ 0.2 N/25 mm/(g/m$^2$), and the accumulated value of stress integration values at 50% elongation of the fabric is $\leq$ 70 N/(g/m$^2$).

8. A hygienic material, comprising the fabric of any of claims 1-7.

**Patentansprüche**

1. Gewebe, das ein versponnener Vliesstoff ist, bestehend aus einer Zusammensetzung, umfassend:

    (1) ein Propylen-Homopolymer mit einem Schmelzpunkt von $\geq$ 140 °C,
    (2) ein Ethylen-Homopolymer mit einer Dichte von 0,941-0,970 g/cm$^3$ und
    (3) mindestens ein Polymer, ausgewählt aus (I) und (II):

    (I) ein beliebiges Copolymer, das eine von Propylen abgeleitete Struktureinheit enthält und mindestens eine Struktureinheit, ausgewählt aus einer von Ethylen abgeleiteten Struktureinheit und einer von einem $C_{4-20}$-$\alpha$-Olefin abgeleiteten Struktureinheit, das einen Schmelzpunkt von $\geq$ 130°C aufweist und das eine Schmelzflussrate von 30-70 g/10 min aufweist, wobei der Schmelzpunkt definiert ist als die Peakspitze eines Peaks, der auf der Seite der höchsten Temperatur einer schmelzendothermen Kurve zu sehen ist, die unter Verwendung eines Differentialscanningkalorimeters (DSC) erhalten wird, bei dem ein Polymer für 5 Minuten bei einer Stickstofftemperatur von -40 °C aufbewahrt wird und die Temperatur dann um 10 °C/min erhöht wird und die Schmelzflussrate mit einem Verfahren gemäß ASTM-Standard D-1238 bei 230 °C, 2160 g Beschickung gemessen wird und
    (II) ein Propylenhomopolymer mit einem Schmelzpunkt von < 120°C, wobei der Schmelzpunkt definiert ist als die Peakspitze eines Peaks, der auf der Seite der höchsten Temperatur einer schmelzendothermen Kurve zu sehen ist, die unter Verwendung eines Differentialscanningkalorimeters (DSC) erhalten wird, bei dem ein Polymer für 5 Minuten bei einer Stickstofftemperatur von -10 °C aufbewahrt wird und die Temperatur dann um 10 °C/min erhöht wird,
    und das folgende Bedingungen (a)-(f) erfüllt:

    (a) [mmmm] = 20-60 mol-%
    (b) [rrrr]/(1-[mmmm]) $\leq$ 0,1
    (c) [rmrm] > 2,5 mol%
    (d) [mm]$\times$[rr]/[mr]$^2$ $\leq$ 2,0
    (e) gewichtsgemitteltes Molekulargewicht (Mw) = 10.000-200.000, wobei das gewichtsgemittelte Molekulargewicht nach dem in der Beschreibung angegebenen Verfahren gemessen wird
    (f) Molekulargewichtsverteilung (Mw/Mn) < 4, wobei die Molekulargewichtsverteilung nach dem in der Beschreibung angegebenen Verfahren bestimmt wird,

    worin [mmmm] eine Mesopentadenfraktion ist, [rrrr] eine razemische Pentadenfraktion ist, [rmrm] eine razemische Mesopentadenfraktion ist und [mm], [rr] und [mr] jeweils eine Triadenfraktion sind, die nach dem in der Beschreibung angegebenen Verfahren gemessen werden,
    worin der Gesamtgehalt des aus (I) und (II) ausgewählten Polymers, bezogen auf die Gesamtmenge der Zusammensetzung, 5-30 Massen-% beträgt.

2. Gewebe gemäß Anspruch 1, worin die Zusammensetzung, bezogen auf die Gesamtmenge der Zusammensetzung, 0,1-5,0 Massen-% eines $C_{15-22}$ Fettsäureamids enthält.

3. Gewebe gemäß Anspruch 1 oder 2, worin die Zusammensetzung, bezogen auf die Gesamtmenge der Zusammensetzung, 55,0-89,9 Massen-% des Propylen-Homopolymers (1) enthält.

4. Gewebe gemäß einem beliebigen der Ansprüche 1-3, worin die Zusammensetzung, bezogen auf die Gesamtmenge der Zusammensetzung, 1,0-10,0 Massen-% des Ethylen-Homopolymers (2) enthält.

5. Gewebe gemäß einem beliebigen der Ansprüche 1-4, worin das Polymer (I) ein Zufallscopolymer aus Propylen und Ethylen ist.

**6.** Gewebe gemäß einem beliebigen der Ansprüche 1-5, worin das Polymer (I) ein Zufallscopolymer aus Propylen, Ethylen und 1-Buten ist.

**7.** Gewebe, gemäß einem beliebigen der Ansprüche 1-6, worin ein Wert, der durch Teilen der 5% Festigkeit des Gewebes durch dessen Flächengewicht erhalten wird, $\geq 0{,}2$ N/25 mm/(g/m$^2$) beträgt und der kumulierte Wert der Spannungsintegrationswerte bei 50% Dehnung des Gewebes $\leq 70$ N/ (g/m$^2$) beträgt.

**8.** Hygienematerial, das das Gewebe gemäß mindestens einem der Ansprüche 1-7 umfasst.


**Revendications**

**1.** Tissu qui est un tissu non tissé par filage direct, constitué d'une composition comprenant

(1) un homopolymère de propylène présentant un point de fusion $\geq 140$ °C,
(2) un homopolymère d'éthylène présentant une densité de 0,941 à 0,970 g/cm$^3$, et
(3) au moins un polymère sélectionné parmi (I) et (II) :

- (I) un copolymère aléatoire qui inclut un motif constitutif dérivé du propylène et au moins un motif constitutif sélectionné parmi un motif constitutif dérivé de l'éthylène et un motif constitutif dérivé d'une $\alpha$-oléfine en C$_{4\text{-}20}$, qui a un point de fusion $\geq 130$ °C, et qui a un indice de fluidité à chaud de 30 à 70 g/10 min, le point de fusion étant défini comme le sommet d'un pic observé du côté de la température la plus élevée d'une courbe endothermique de fusion obtenue à l'aide d'un calorimètre différentiel à balayage (DSC) dans lequel un polymère est maintenu dans une atmosphère d'azote à -40 °C pendant 5 min, puis la température est élevée à 10 °C/min et l'indice de fluidité à chaud est mesuré par un procédé selon la norme ASTM D-1238 à 230 °C, charge 2160 g et
- (II) un homopolymère de propylène présentant un point de fusion < 120 °C, le point de fusion étant défini comme le sommet d'un pic observé du côté de la température la plus élevée d'une courbe endothermique de fusion obtenue en utilisant un calorimètre différentiel à balayage (DSC) dans lequel un polymère est maintenu dans une atmosphère d'azote à -10 °C pendant 5 min, puis la température est élevée à 10 °C/min, et satisfaisant les (a) à (f) suivants :

(a) [mmmm] = 20 à 60 % en mole
(b) [rrrr]/(1-[mmmm]) $\leq 0{,}1$
(c) [rmrm] > 2,5 % en mole
(d) [mm]$\times$[rr]/[mr]$^2 \leq 2{,}0$
(e) masse moléculaire moyenne en poids (Mw) = 10 000 à 200 000, la masse moléculaire moyenne en poids étant mesurée selon le procédé de la description
(f) distribution de poids moléculaire (Mw/Mn) < 4, la distribution de poids moléculaire étant déterminée selon le procédé de la description,

où [mmmm] est une fraction méso pentade, [rrrr] est une fraction pentade racémique, [rmrm] est une fraction pentade méso racémique méso racémique, et [mm], [rr] et [mr] sont chacun une fraction de triade mesurée selon le procédé de la description, et

dans lequel la teneur totale du polymère sélectionné parmi (I) et (II), sur la base de la quantité totale de la composition, est de 5 à 30 % en masse.

**2.** Tissu selon la revendication 1, dans lequel la composition contient, sur la base de la quantité totale de la composition, 0,1 à 5,0% en masse d'un amide d'acide gras en C$_{15\text{-}22}$.

**3.** Tissu selon la revendication 1 ou 2, dans lequel la composition contient, sur la base de la quantité totale de la composition, 55,0 à 89,9 % en masse de l'homopolymère de propylène (1).

**4.** Tissu selon l'une quelconque des revendications 1 à 3, dans lequel la composition contient, sur la base de la quantité totale de la composition, 1,0 à 10,0 % en masse de l'homopolymère d'éthylène (2).

**5.** Tissu selon l'une quelconque des revendications 1 à 4, dans lequel le polymère (I) est un copolymère aléatoire de propylène et d'éthylène.

**6.** Tissu selon l'une quelconque des revendications 1 à 5, dans lequel le polymère (I) est un copolymère aléatoire de propylène, d'éthylène et de 1-butène.

**7.** Tissu selon l'une quelconque des revendications 1 à 6, dans lequel une valeur obtenue en divisant la résistance à 5 % du tissu par son poids de base est $\geq 0,2$ N/25 mm/(g/m$^2$), et la valeur accumulée des valeurs d'intégration de contrainte à 50 % d'allongement du tissu est $\leq 70$ N/(g/m$^2$).

**8.** Matériau hygiénique, comprenant le tissu selon l'une quelconque des revendications 1 à 7.

## FIG.1

FIG.2

# FIG.3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H07503502 A
- JP 3798018 B
- WO 2014050965 A1
- JP 2015071854 A
- US 20100124864 A1
- WO 2003087172 A

**Non-patent literature cited in the description**

- **A. ZAMBELLI et al.** *Macromolecules,* 1973, vol. 6, 925
- **A. ZAMBELLI et al.** *Macromolecules,* 1975, vol. 8, 687